Europäisches Patentamt

European Patent Office

Office européen des brevets

(11) Publication number: **0 092 299**
**B1**

(12) **EUROPEAN PATENT SPECIFICATION**

(45) Date of publication of patent specification: **05.08.87**

(21) Application number: **83300552.3**

(22) Date of filing: **03.02.83**

(51) Int. Cl.⁴: **A 61 F 5/44**

(54) Fold over filter clip and ostomy bag for use with same.

<table>
<tr><td>

(30) Priority: **15.02.82 GB 8204410**
**12.05.82 GB 8213811**
**27.07.82 GB 8221693**
**27.07.82 GB 8221660**
**30.09.82 GB 8227877**

(43) Date of publication of application:
**26.10.83 Bulletin 83/43**

(45) Publication of the grant of the patent:
**05.08.87 Bulletin 87/32**

(84) Designated Contracting States:
**AT BE CH DE FR GB IT LI LU NL SE**

(56) References cited:
**EP-A-0 023 781**
**GB-A-1 550 960**
**GB-A-2 031 282**
**GB-A-2 058 011**

</td><td>

(73) Proprietor: **CRAIG MEDICAL PRODUCTS LIMITED**
**Claygate House 46 Albert Road North**
**Reigate Surrey RH2 9EN (GB)**

(72) Inventor: **Steer, Peter Leslie**
**The Malt House Works Station Road**
**East Grinstead Sussex (GB)**
Inventor: **Edwards, John Victor**
**5, Lodge Close**
**East Grinstead Sussex (GB)**

(74) Representative: **Cook, Anthony John et al**
**D. YOUNG & CO. 10, Staple Inn**
**London, WC1V 7RD (GB)**

</td></tr>
</table>

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Most ostomates employ some type of bag or pouch system to collect bodily wastes discharged from their surgically created stoma. Today, such pouches are generally formed of lightweight, odor-proof, flexible polymeric materials and the collection systems are designed to be inconspicuous and permit the ostomate to engage in normal physical activity. However, many ostomates, particularly immediately following surgery, have fears concerning their ability to resume a "normal life". These fears center around worries that the collection system will leak or that odor will escape and that the system will be noticeable even through their outer clothing. Part of these problems are due to the discharge of flatus into the pouch which can cause an embarrassing distention of the pouch.

EP—A—23781 (KINGSDOWN) discloses an attachable and detachable filter for use with an ostomy bag, includes a plastics housing member having two portions hinged together by an integral hinge, each portion having a peripheral wall and at least one of the walls having apertures therein. One portion of the housing member carried a sharp-pointed and resilient-headed generally cylindrical stud which is a push fit through an aperture in the other to clamp together the two portions, there being at least one layer of gas-filtering material contained within one or other or both the housing portions.

GB—A—1 550 960 (SUZUKI) discloses a deodorising adhesive seal for treating odorous gas, e.g. arising from excrement in an ostomy bag. The seal disclosed comprises a layer of gas-permeable material containing or consisting of an absorbent substance capable of absorbing odorous gas and having a layer of gas-impermeable material on one side thereof and a gas-permeable layer of adhesive on the other side thereof.

GB—A—2 031 282 discloses a surgical collection bag for post operative use having two opposed walls, secured together by a perimeter weld. One of the walls has an inlet opening through which the contents of the intestine of a patient can flow into the interior of the bag. The same wall also has a vent which is impervious to solid material and liquid from the intestine of the patient but which is pervious to gases. Welds inside the bag together with the perimeter weld define a pathway through which gases can flow from the interior of the bag to the vent. This pathway includes one or more gateways through which gases must pass before they reach the vent. These gateways are sufficiently narrow to prevent the passage of solid materials and liquids. If desired, the welds can be discontinuous to provide a multiplicity of gateways.

This invention relates to a filter clip which can be readily attached to an ostomy bag. This invention also includes an ostomy bag particularly suited for use with an attachable filter. The term "ostomy bag" is used to means a colostomy or ileostomy or other kind of bag or pouch intended to be worn by a user to receive waste material expelled from a stoma.

It would be desirable to have a clip-on filter which can be manufactured in a simple and inexpensive manner and can be easily and securely attached to an ostomy bag, even by elderly or infirm persons, which does not require a hole to be punched in the bag wall in a separate preliminary operation, and, when once attached, is so constructed that gases leaving the bag are required to take a path of reasonable length through gas filtering material.

According to the invention, there is provided a fold-over filter clip for use on an ostomy bag comprising first and second members joined together or hinged together and made from a material which can be readily bent by human finger pressure, said first member having a bag puncturing spike extending outwardly from a substantially flat surface thereon and said second member defining a space in which a filter pad of gas adsorbing material is located, the second member having a base wall and having one or more small apertures through which filtered gas can egress, characterised by respective layers of adhesive each covered by a strippable protective layer provided on the substantially flat surface of the first member and on a flat surface extending across the top of the second member except in the area which aligns with said spike when the clip is folded over into its closed position.

Also according to the invention, there is provided an ostomy bag comprising front and rear walls of synthetic plastics material sealed together along at least a substantial portion of their peripheral edges, an opening in said front bag wall through which the stoma can protrude, the bag having an upper bag region above said stomal opening defined by a discontinuous arcuate join which connects said front and rear bag walls, said join being such as to permit gas to flow past the join through a plurality of breaks therein but such as to impede the flow of fecal material, said discontinuous join being formed by heat welding the front and rear bag walls together, characterised in that short bar welds are located adjacent to and across each gap to further define a labyrinthine path for gases passing from the lower part of said bag into said upper bag region.

Further, according to the invention, there is provided an ostomy bag having an attached fold over filter clip, said ostomy bag comprising front and rear walls of synthetic plastics material sealed together along at least a substantial portion of their peripheral edges, an opening in said front bag wall through which the stoma can protrude, the bag having an upper bag region above said stomal opening defined by a discontinuous join which connects said front and rear bag walls, said join being such as to permit gas to flow past the join through one or more breaks therein but such as to impede the flow of fecal material; a fold over filter clip attached to the ostomy bag in upper bag region said filter clip comprising first and second members joined together or hinged together and made from a material which can be readily bent by human finger pressure, said first member having a bag puncturing spike, said second member including a base wall and having one or more small apertures through which filtered gas can egress and which defines a space in which a filter pad of gas adsorbing material is located, characterised by the spike extending outwardly from a substantially flat surface on the first member and having an adhesive covered by a strippable protective layer on said flat surface and by a layer of adhesive covered by a strippable protective layer extending across the top of said second member except in the area which aligns with said spike when the clip is folded over into its closed position, the management being such that said filter clip is attached to said upper bag region by removing said strippable protective layers, locating said filter clip at said upper bag region and in one step bending and pressing said filter clip so that adhesive contact is made between said front and rear bag walls and said first and second clip members while at the same time said spike is puncturing both of said bag walls.

In use, a clip as defined above is bent over into U-shape embracing the top edge of an ostomy bag, after the strippable protective layers have been removed from the adhesive, and its two members are pressed together gripping a portion of the bag between them, so that the clip is securely held on the bag by the two adhesive layers. In this folding-over operation, the spike punctures both the front and the rear wall of the ostomy bag and the construction of the filter is such that exiting gases pass through the filter pad. That is to say, they pass from the filter pad into the ambient atmosphere after having been effectively filtered.

The two hinged or joined members are preferably molded with an integral hinge from a moldable synthetic plastics material such as polypropylene. Alternatively the two may be made from a single piece of metal such as aluminium or an aluminium alloy. The function of the members is to carry the filter pad and the spike respectively and any material which can be readily bent by finger pressure can be used. The spike can be integral with the first plate member or may be fixed thereto in a convenient manner.

In a preferred embodiment of the invention, a controlled porosity layer is located in juxtaposition with the filter pad, on the side of the filter pad which in use will be nearer the bag. Such a controlled porosity layer may be made from an open-cell foamed synthetic plastics material. It may also be termed a microporous membrane. Its function is to offer a controlled impediment to gases leaving the ostomy bag and passing into the filter pad, so that the gas pressure in the interior of the bag will not fall suddenly to such a level that the front and rear bag walls collapse into contact with each other. If this occurs, the wearer may suffer pain or discomfort as the outer bag wall contacts the sensitive end of the stoma, and free discharge of material from the stoma may be hindered. At the same time, the porosity of the layer is chosen so that the obstruction to escaping gases offered by the layer is not so great as to prevent adequate venting of flatus gases from the bag interior.

This invention is also directed to an ostomy bag particularly adapted for use with an attachable filter. One problem encountered with ostomy bags to which filters are attached, in whatever manner, is that liquid or slurry-like fecal material within the bag may be brought into the region of the exit aperture to the filter. This may easily occur, for example, if the bag is accidentally squeezed or compressed while being worn. In consequence, the filter material may be contamined and its pores may be occluded.

According to the present invention, there is provided an ostomy bag characterized in that an upper region thereof is defined by a discontinuous join which connects the front and rear wall of the bag, the join being such as to permit gas to flow past the join through one or more breaks therein but such as to impede flow of liquid of slurry-like fecal material.

In a preferred embodiment of the invention, the join is formed by heat-welding the front and rear walls together. Such a weld may be arcuate in form and may have one or more gaps therein. Optional, each gap may be partly obstructed by a short bar weld spaced therefrom to define a labyrinthine path for gases passing from the remainder of the interior of the bag (i.e., its lower part) into the aforesaid upper region.

Figure 1 is a plan view of a filter clip according to one example of the invention showing two hinged members, seen in the open position;

Figure 2 is an underplan view corresponding to Figure 1;

Figure 3 is a vertical central cross-section through the filter clip of Figure 1;

Figure 4 is a side elevation of the filter clip of Figure 1;

Figure 5 is a section on A—A in Figure 2;

Figure 6 is a front elevation of an ostomy bag according to this invention having a filter clip according to the invention attached thereto;

Figure 7 is a top plan view of a filter clip according to a second embodiment of the invention shown in the open condition;

Figure 8 is an underplan view corresponding to Figure 7;

Figure 9 is a vertical central cross-section through the clip shown in Figure 7;

Figure 10 is a side elevation of a clip according to Figures 7—9 but showing the space containing the filter pad closed;

Figure 11 is a cross-section on the line A—A of Figure 8; and

Figure 12 is a front elevation of an ostomy bag according to another embodiment of this invention having a filter clip according to the invention attached thereto.

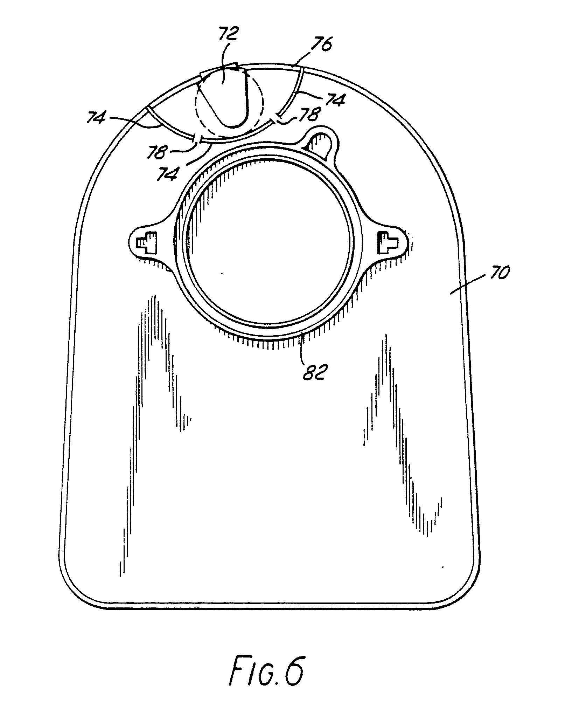

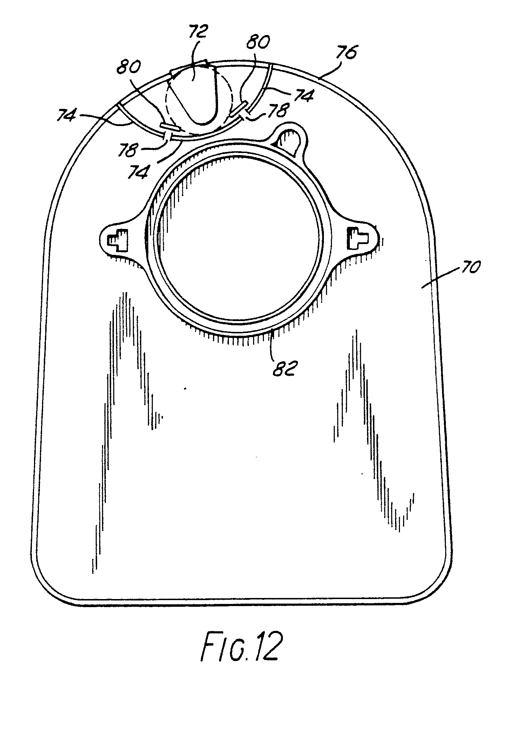

Modern ostomy bags, as shown in Figures 6 and 12, are made of two superposed sheets of synthetic plastics material welded around their edges. The walls of the bag are of synthetic plastics material, and are attached together by a weld seam around the bag. A fold-over filter clip according to the invention is folded over an upper region of the bag. This clip has a first member and a second member. The first member carries a spike and this spike is surrounded by an adhesive on an inner surface of the first member. In its folded-over position the clip may be pinched or pressed onto the top of the ostomy bag in such a way that the spike punctures both bag walls.

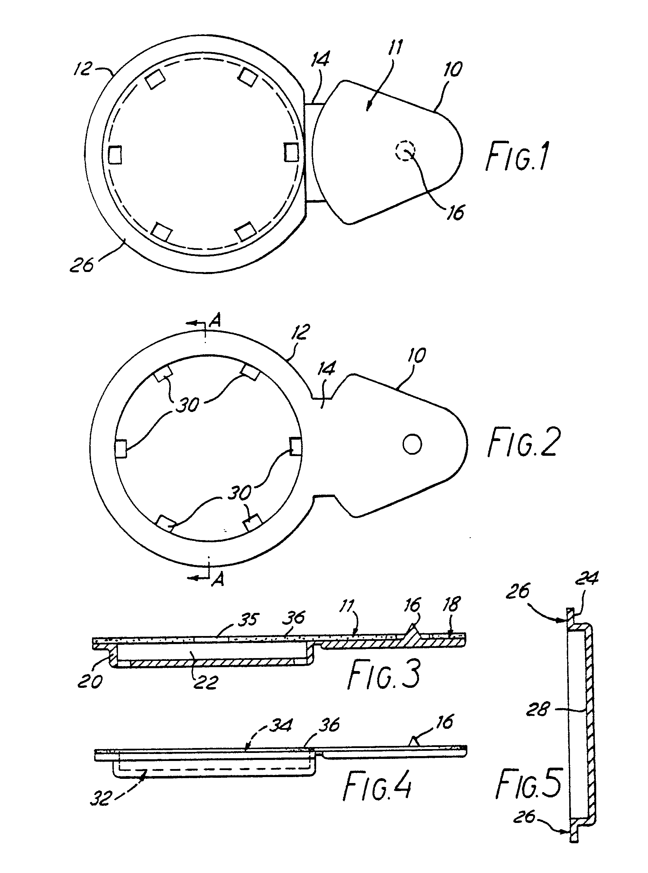

The filter clip illustrated in Figures 1—5 includes a first member hinged to a second member 12 by a hinge 14. The first member 10 carries (or is integral with) a spike 16 which extends from a flat surface 18. The second member 12 has a peripheral wall 20 defining a space 22 which is the shape of a flat cylindrical disc. The wall 20 has an integral flange 24 which presents an upwardly-facing flat surface 26. The space 22 is bounded by a base wall 28 which may be integral with the wall 20 or may be a removable plate which is a push fit into the second member. The wall or plate 28 has a series of cut outs or holes 30 around its outer portion. The function of these holes or cut outs is to allow exit of filtered gases from a disc-shaped filter pad which is placed in the space 22.

The flat surface 26 and the upper surface 34 of the pad 32 (shown dotted only in Figure 4) is covered (except for a central hole 35) with a layer 36 of latex adhesive which has on its top surface (as seen in Figures 3 and 4) a layer of strippable protective paper or film, which may for example be silicone treated to permit ready release.

The flat surface 11 of the member 10 surrounding the spike is similarly covered. The adhesive and strippable layer covering flat surface 11 may be contiguous with the adhesive and strippable layer covering the surfaces 26 and 34.

In use, the user strips the protective layer from the adhesive and folds the clip over the top edge of an ostomy bag in the manner illustrated in Figures 6 and 12. The spike 16 punctures both bag walls, and the first member 10 is attached to the outer surface of one bag wall by the adhesive which entirely surrounds the spike. Consequently any gases which may escape through the spike hole cannot escape further because of the circular area of adhesive attaching the said one bag wall to the surface 11 of the member 10. Gases can, however, exit through the hole punched by the spike 16 in the other bag wall, as the circular area 35 is free of adhesive, so exposing a central region of the disc shaped filter pad 32. The gases then pass into the center of the pad 32 and move radially outwardly therein to the exit holes 30, and in so doing are filtered and deodorized by the action of the activated carbon or like material of the pad 32. In this way, a relatively lengthy path for escape and filtering of flatus gases is achieved.

In the embodiment of the invention which employs a base wall 28 integral with the wall 20 and the flange 24, the clip can be made in a single molding operation, the disc filter pad can then be inserted into the space 22 and one single further operation suffices to apply the strippable layer already cut to shape and already coated with adhesive.

In the embodiment of the invention which employs a snap-fitted removable plate 28 to provide the base wall of the space 22, the parts 10, 12, 16, 20, 24 are integrally molded, the adhesive and strippable layer are applied thereto with the latter layer lowermost, the disc filter pad is dropped into the space 22 and the plate 28 is snap fitted to hold the pad 32 within the second member 12.

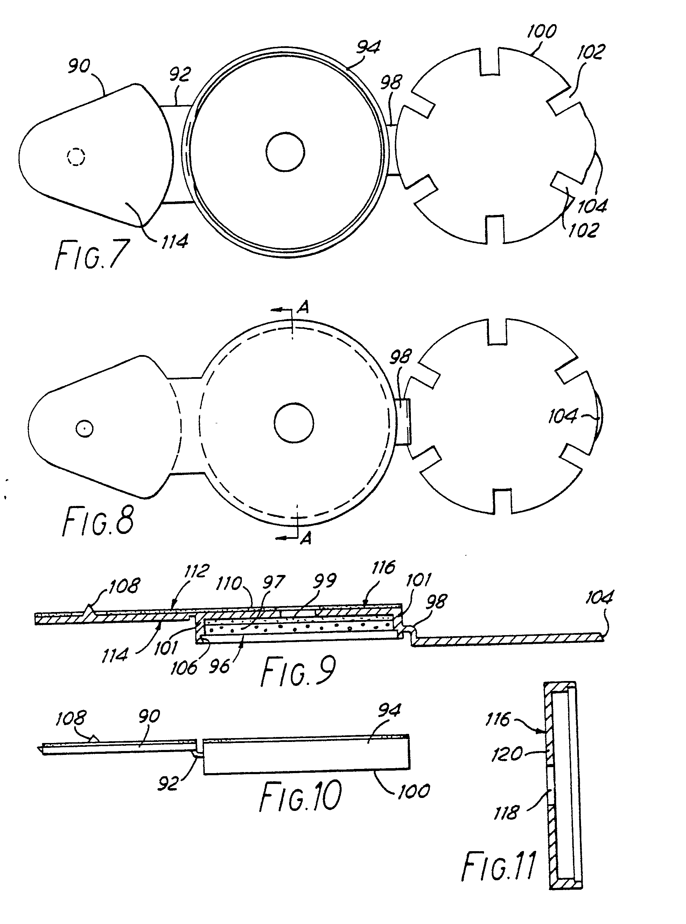

An alternative embodiment of the filter clip of this invention is shown in Figures 7—11. The filter clip shown therein is similar in principle to that shown in Figures 1—5. It has a first member 90 joined by an integral hinge 92 to a second member 94 which defines a space 96 to receive a filter pad 97 and a controlled porosity layer 99. The second member 94 is joined by an integral hinge 98 to a lid 100 which has a number of slots 102 in its periphery. As shown, the second member and lid are circular in shape, but of course this is not essential; they could, for example, be oval. The lid has a rim portion 104 diametrically opposite to the hinge 98 which cooperates with a catch 106 formed by an inclined surface on the inner rim of the second member 94.

Due to this construction, the lid can be snapped into its closed position, once the filter 97 and the controlled porosity layer 99 have been placed in the space 96 during assembly of the filter clip. The layer 99 may be adhesively sealed at its rim to adjacent side wall 101 of the second member 94. The first member 90 has a spike 108 and a layer of adhesive 110, itself covered by a strippable protective layer 112, which covers surfaces 114, 116 of the first and second member in a similar manner, and for a similar purpose, to the corresponding layers described with reference to Figures 1—5. A hole 118 (not covered by layers 110 and 112) is provided in the upper wall 120 of the second member 94 to allow the flatus gases to pass into the central region of the filter pad. The filtering action, and the manner of application to an ostomy bag, of this embodiment of the invention are in essence the same as those of the embodiment shown in Figures 1—5, apart from the advantages provided by the controlled porosity layer 99. Of course, this controlled porosity layer could be employed in the filter clip embodiment of Figures 1—5 between the upper surface 34 of pad 32 and layer 36.

The filter pads 32 and 97 preferably contain activated carbon as the gas adsorbing and deodorizing agent. One type of suitable material is a sheet of foamed open-cell non-woven synthetic polymeric material, for example, polyurethane, having a large number of activated carbon particles distributed over one of its major surfaces. Such a material is commercially available under the tradename Bondina. Another type of suitable deodorizing material is a felt pad impregnated with activated carbon in fine particulate form. Various types of such carbon cloth are commercially available.

Preferably, the filter pad is made by cutting discs from a sheet of filter medium known as "Bondina" activated carbon filter No. S.442. Tests on one inch diameter discs of this filter material have shown it to be highly successful in deodorizing a gas mixture consisting of 20% methane, 25 p.p.m. H S and the balance nitrogen.

Preferably, the controlled porosity layer is known as "Bondina" non-woven viscose medical tape, No. T.1562 F. This material has a thickness of 0.23 mm and a weight of 55 g/m². It is permeable to air at 480 l/minute, 0.5 m bar DIN 53 887—77.

The slots 102 shown around the periphery of lid 100 may be replaced by a series of small apertures placed slightly inward from the peripheral edge of lid 100.

It is generally intended that the fit between hinge 104 and catch 106 be permanent. If desired, this can be ensured by use of adhesive or by spot welding. However, in most instances the force of the snap fit is itself sufficient. Of course, it is also possible to provide means whereby hinge 104 can be released from catch 106 to permit replacement of filter pad 97 and porosity layer 99 with a fresh refill.

In either embodiment, the manufacture and assembly of the filter clip is simple and relatively inexpensive, and in operation the adhesive sealing of the clip surfaces to the outside of the bag walls ensures that there is no exit path available for flatus gases that does not involve a lengthy passage through the filtering material. The provision of a spike so that the user automatically punctures the bag correctly as he or she applies the clip and then in the same movement of the fingers achieves an adhesive seal as described, is a particularly important and useful feature for wearers who are old or infirm and find difficulty in manipulation.

It has been found that elderly patients or patients in poor health find it difficult to satisfactorily apply a conventional patch-filter such as that shown in British Patent 1,550,960 or U.S. Patent 3,952,727.

Figures 6 and 12 shown an ostomy bag 70 according to this invention. The illustrated ostomy bags are conventional except that the front and rear walls are welded together with arcuate welds 74. These welds make up a curved weld joint joining the walls and whose two ends extend to the top edge weld seam 76 of the bag. At least one gap (in fact two gaps 78 are shown) is provided between the arcuate welds so that flatus gases can enter the space bounded by the curved weld joint, the edge weld, and the front and rear walls of the bag. In use, the spike of the fold over filter clip 72 of this invention is used to puncture the front and rear bag walls within the space referred to. The function of the curved weld joint is to prevent fecal matter reaching the hole punched by the spike. As seen in the drawing, two gaps are provided separating three arcuate welds, but any convenient number may be used. The width of each gap 78 has dimensions chosen so that the gap offers a desired resistance to flow of gas from the lower part of the bag to the upper space. Each gap 78 defines a path which allows gas to pass from the remainder of the bag into the upper space demarcated by welds 74, but inhibits or substantially prevents fecal matter passing into the upper space. This avoids choking of the filter pad with slurry-like bag contents, and tends to prevent the bag walls collapsing towards each other.

The width of each gap 78 is preferably between about 0.1 inch (2.5 mm) and about 0.14 inch (3.6 mm) and the gaps may be spaced at equal intervals along the weld. The weld 74 need not be arcuate, it could for example define a part-rectangular space.

The embodiment of the ostomy pouch shown in Figure 12 includes bar welds 80 located across each gap 78. The bar welds 80 in conjunction with the welds 74 define a labyrinthine path at each gap which allows gas to pass readily from the remainder of the bag into the upper region demarcated by welds 74, but further inhibits or substantially prevents fecal matter passing into the upper region.

The ostomy bags illustrated include a channel shaped coupling 82 adapted to receive a rib shaped coupling projecting outwardly from an adhesive pad attached to the body around the stoma. Coupling member 82 surrounds an aperture in the front bag wall through which the stoma can protrude. Such a coupling system and ostomy pouch are described in more detail by Steer et al. in British Patents 1,571,657 and 1,586,824. Of course, the inclusion of channel shaped coupling member 82 is an optional feature on the ostomy bag of this invention. If desired, an adhesive faceplate could be employed in place of coupling member 82 in order to attach the bag directly to the body as note, for example, U.S. Patent 3,055,368 of Baxter and U.S. Patent 4,185,630 of Neumeier et al.

Also, while the ostomy bag 70 is shown as having a closed end, i.e., weld 76 extends around the entire periphery of the bag walls, the limited access upper region feature of this invention may be employed with an otherwise conventional drainable bag such as that shown, for example, by Nolan in U.S. Patent 3,523,534.

While there has been described, in connection with Figures 6 and 12, the use of a bag of a particular design with a fold over filter clip according to the present invention, it is important to note that a clip according to the invention can be used with any of the designs of ostomy bags currently on the market. Also, the ostomy bag of this invention can be employed with filters that do not include their own puncturing spike.

## Claims

1. A fold-over filter clip for use on an ostomy bag comprising first and second members (10, 12; 90, 94) joined together or hinged together and made from a material which can be readily bent by human finger pressure, said first member (10; 90) having a bag puncturing spike (16; 108) extending outwardly from a substantially flat surface (18; 114) thereon and said second member (12; 94) defining a space (22; 96) in which a filter pad (32; 94) of gas adsorbing material is located, the second member having a base wall (28; 100) and having one or more small apertures (30; 102) through which filtered gas can egress, characterised by respective layers of adhesive (36; 110) each covered by a strippable protective layer provided on the substantially flat surface (18; 114) of the first member (10; 90) and on a flat surface extending across the top of the second member (12; 94) except in the area which aligns with said spike (16; 108) when the clip is folded over into its closed position.

2. A clip according to Claim 1 in which said first and second members are moulded with an integral hinge (14; 92) from a mouldable synthetic plastics material.

3. A clip according to Claim 2 in which said second member (12) comprises a peripheral wall (20) having an integral flange (24) which presents an upwardly facing flat surface (26) and the base wall (28) integral with said peripheral wall thereby defining the space (22) in which a filter pad (32) of gas adsorbing material is located, said base wall having one or more small apertures (30) through which filtered gas can egress.

4. A clip according to Claim 3 in which a layer of controlled porosity material is interposed between the top of said filter pad and said layer of adhesive.

5. A clip according to Claim 2 in which said second member (94) comprises an upper wall (115) having a centrally located opening (118) which aligns with the spike (108) of said first member (90) when the clip is bent into its closed position, a side wall (101) integral with said upper wall and extending downwardly from the periphery of said upper wall, and a lid (100) having one or more relatively small apertures (102), said lid being integrally connected by a hinge (98) to one section of said side wall whereby said lid can be snapped into a locked position against the opposite section (106) of said side wall thereby defining a space (96) between said upper wall, said side wall, and said lid; a filter pad (97) of gas adsorbing material located in said space; said first member being connected to said second member upper wall by the integral hinge (92), and the layer of adhesive (110) covered by a strippable protective layer (112) extending across the top of said upper wall except in the area of said upper wall opening.

6. A clip according to any preceding claim in which a layer (99) of controlled porosity material is interposed between said filter pad and said second member upper wall.

7. A clip according to any preceding claim in which said filter pad contains activated carbon.

8. An ostomy bag comprising front and rear walls of synthetic plastics material (76) sealed together along at least a substantial portion of their peripheral edges, an opening in said front bag wall through which the stoma can protrude, the bag having an upper bag region above said stomal opening defined by a discontinuous arcuate join (74) which connects said front and rear bag walls, said join being such as to permit gas to flow past the join through a plurality of breaks (78) therein but such as to impede the flow of fecal material, said discontinuous join being formed by heat welding the front and rear bag walls together, characterised in that short bar welds (80) are located adjacent to and across each break to further define a labyrinthine path for gases passing from the lower part of said bag into said upper bag region.

9. The ostomy bag of Claim 8 in which a channel shaped coupling member (82) is located on the front bag wall surrounding said stomal opening.

10. An ostomy bag having an attached fold over filter clip, said ostomy bag comprising front and rear walls of synthetic plastics material sealed together along at least a substantial portion of their peripheral edges, an opening in said front bag wall through which the stoma can protrude, the bag having an upper bag region above said stomal opening defined by a discontinuous join (74) which connects said front and rear bag walls, said join being such as to permit gas to flow past the join through one or more breaks (78) therein but such as to impede the flow of fecal material; a fold over filter clip (72) attached to the ostomy bag in upper bag region said filter clip comprising first and second members joined together or hinged together and made from a material which can be readily bent by human finger pressure, said first member having a bag puncturing spike, said second member including a base wall and having one or more small apertures through which filtered gas can egress and which defines a space in which a filter pad of gas adsorbing material is located, characterised by the spike extending outwardly from a substantially flat surface on the first member and having an adhesive covered by a strippable protective layer on said flat surface and by a layer of adhesive covered by a strippable protective layer extending across the top of said second member except in the area which aligns with said spike when the clip is folded over into its closed position, the management being such that said filter clip is attached to said upper bag region by removing said strippable protective layers, locating said filter clip at said upper bag region and in one step bending and pressing said filter clip so that adhesive contact is made between said front and rear bag walls and said first and second clip members while at the same time said spike is puncturing both of said bag walls.

**Patentansprüche**

1. Faltbare Filterklammer zur Verwendung auf einem Ostomiebeutel, mit einem ersten und einem zweiten Bauteil (10, 12; 90, 94), die miteinander verbunden oder aneinander angelenkt sind und aus einem Material bestehen, das durch menschlichen Fingerdruck leicht biegbar ist, wobei das erste Bauteil (10; 90) eine von einer im wesentlichen ebenen Oberfläche (18; 114) des Bauteils (10; 90) sich nach außen erstreckende Spitze (16; 108) zum Durchstechen des Beutels aufweist und das zweite Bauteil (12; 94) einen Raum (22; 96) begrenzt, in dem ein Filterkissen (32; 94) aus gasadsorbierendem Material angeordnet ist, und wobei das zweite Bauteil eine Bodenwand (28; 100) und eine oder mehrere kleine Öffnungen (30; 102) aufweist, durch die gefiltertes Gas entweichen kann, gekennzeichnet durch jeweils von einer abziehbaren Schutzschicht bedeckte Kleberschichten (36; 110), die auf der im wesentlichen ebenen Oberfläche (18; 114) des ersten Bauteils (10; 90) und auf einer sich über die Oberseite des zweiten Bauteils (12; 94) erstreckenden ebenen Oberfläche angeordnet sind, mit Ausnahme des Bereiches, der mit der Spitze (16; 108) ausgerichtet ist, wenn die Klammer in ihre geschlossene Stellung umgefaltet ist.

2. Klammer nach Anspruch 1, dadurch gekennzeichnet, daß das erste und zweite Bauteil mit einem einstückigen Gelenk (14; 92) aus einem gießbaren synthetischen Kunststoffmaterial gegossen sind.

3. Klammer nach Anspruch 2, dadurch gekennzeichnet, daß das zweite Bauteil (12) eine Umfangswand (20) mit einem einstückigen Flansch (24) aufweist, der eine nach oben gerichtete ebene Oberfläche (26) darstellt, und die Bodenwand (28) einstückig mit der Umfangswand ist und dadurch der Raum (22) begrenzt wird, in dem ein Filterkissen (32) aus gasadsorbierendem Material angeordnet ist, wobei die Bodenwand eine oder mehrere kleine Öffnungen (30) aufweist, durch die gefiltertes Gas entweichen kann.

4. Klammer nach Anspruch 3, dadurch gekennzeichnet, daß eine Schicht aus Material mit gesteuerter Porosität zwischen der Oberseite des Filterkissens und der Kleberschicht angeordnet ist.

5. Klammer nach Anspruch 2, dadurch gekennzeichnet, daß das zweite Bauteil (94) aufweist: eine obere Wand (116) mit einer mittig angeordneten Öffnung (118), die mit der Spitze (108) des ersten Bauteils (90) ausgerichtet ist, wenn die Klammer in ihre geschlossene Stellung gebogen ist, eine Seitenwand (101), die mit der oberen Wand einstückig ist und sich vom Umfang der oberen Wand nach unten erstreckt, und einen Deckel (100) mit einer oder mehreren relativ kleinen Öffnungen (102), der einstückig mittels eines Gelenks (98) mit einem Abschnitt der Seitenwand verbunden ist, wodurch der Deckel in eine geschlossene Stellung gegen den gegenüberliegenden Abschnitt (106) der Seitenwand schnappbar ist, wodurch ein Raum (96) zwischen der oberen Wand, der Seitenwand und dem Deckel ausgebildet wird; ein in dem Raum angeordnetes Filterkissen (97) aus gasadsorbierendem Material; wobei das erste Bauteil mittels des einstückigen Gelenks (92) mit der oberen Wand des zweiten Bauteils verbunden ist, und sich die von einer abziehbaren Schutzschicht (112) bedeckte Kleberschicht (110) über die Oberseite der oberen Wand erstreckt mit Ausnahme des Bereiches der Öffnung der oberen Wand.

6. Klammer nach einem der Ansprüche 1 bis 5, dadurch gekennzeichnet, daß eine Schicht (99) aus einem Material mit gesteuerter Porosität zwischen dem Filterkissen und der oberen Wand des zweiten Bauteils angeordnet ist.

7. Klammer nach einem der Ansprüche 1 bis 6, dadurch gekennzeichnet, daß das Filterkissen Aktivkohle enthält.

8. Ostomiebeutel mit einer Vorderwand und einer Rückwand aus synthetischem Kunststoffmaterial (76), die mindestens entlang eines wesentlichen Teils ihrer Umfangskanten dicht miteinander verbunden sind, und einer Öffnung in der Vorderwand des Beutels, durch die das Stoma vorragen kann, wobei der Beutel einen oberen Beutelbereich oberhalb der Stomaöffnung aufweist, der durch eine diskontinuierliche, gekrümmte Verbindung (74) begrenzt ist, die die Vorder- und Rückwand des Beutels miteinander verbindet, wobei die Verbindung derart ausgebildet ist, daß Gas durch mehrere Unterbrechungen (78) durch die Verbindung fließen kann, aber die Strömung von Fäkalien gehemmt wird, wobei die diskontinuierliche Verbindung durch Verschweißen der Vorder- und Rückwand des Beutels unter Wärme ausgebildet wird, dadurch gekennzeichnet, daß in der Nähe und quer zu jeder Unterbrechung kurze, stabförmige Schweißnähte (80) angeordnet sind, so daß für vom unteren Teil des Beutels in den oberen Teil des Beutels strömende Gase ein labyrinthförmiger Weg ausgebildet wird.

9. Ostomiebeutel nach Anspruch 8, dadurch gekennzeichnet, daß ein kanalförmiges Kupplungsteil (82) um die Stomaöffnung auf der Vorderwand des Beutels ausgebildet ist.

10. Ostomiebeutel mit einer daran befestigten faltbaren Filterklammer, dadurch gekennzeichnet, daß der Ostomiebeutel aufweist: eine Vorder- und eine Rückwand aus synthetischem Kunststoffmaterial, die mindestens entlang eines wesentlichen Teiles ihrer Umfangskanten miteinander verbunden sind, eine Öffnung in der Vorderwand des Beutels, durch die das Stoma vorragen kann, wobei der Beutel einen oberen Beutelbereich oberhalb der Stomaöffnung aufweist, der durch eine diskontinuierliche Verbindung (74) begrenzt ist, die die Vorder- und Rückwand des Beutels miteinander verbindet, wobei die Verbindung derart ausgebildet ist, daß Gas durch eine oder mehrere Unterbrechungen (78) durch die Verbindung fließen kann, aber daß die Strömung von Fäkalien gehemmt wird; eine im oberen Beutelbereich an dem Ostomiebeutel befestigte faltbare Filterklammer (72), die erste und zweite Bauteile aufweist, die miteinander verbunden oder aneinander angelenkt sind und aus einem Material bestehen, das durch menschlichen Fingerdruck leicht gebogen werden kann, wobei das erste Bauteil eine Spitze zum Durchstechen des Beutels aufweist und das zweite Bauteil eine Bodenwand und eine oder mehrere kleine Öffnungen aufweist, durch die gefiltertes Gas entweichen kann und einen Raum begrenzt, in dem ein Filterkissen aus gasadsorbierendem Material angeordnet ist, dadurch gekennzeichnet, daß die Spitze sich von einer im wesentlichen ebenen Fläche auf dem ersten Bauteil nach außen erstreckt und ein von einer abziehbaren Schutzschicht bedeckter Kleber auf der ebenen Fläche angeordnet ist, und durch eine Kleberschicht, die von einer abziehbaren Schutzschicht bedeckt ist, die sich über die Oberseite des zweiten Bauteils erstreckt mit Ausnahme des Bereiches, der mit der Spitze ausgerichtet ist, wenn die Klammer in die geschlossene Stellung umgefaltet ist, wobei die Anordnung derart ist, daß die Filterklammer am oberen Beutelbereich befestigt wird durch Entfernen der abziehbaren Schutzschichten, Anordnen der Filterklammer im oberen Beutelbereich, sowie Biegen und Pressen der Filterklammer in einem Schritt, so daß zwischen der Vorder- und Rückwand des Beutels und dem ersten und dem zweiten Bauteil der Klammer ein Klebekontakt ausgebildet wird, während gleichzeitig die Spitze beide Beutelwände durchsticht.

**Revendications**

1. Agrafe de filtre repliable destinée à être utilisée sur une poche de stomie comprenant un premier et un second éléments (10, 12; 90, 94) réunis l'un à l'autre ou articulés l'un sur l'autre et faits d'un matériau qui peut facilement être plié par la pression des doigts d'une personne, ledit premier élément (10; 90) comportant une pointe (16; 108) de perçage de poche faisant saillie à l'extérieur d'une surface sensiblement plate (18; 114) et ledit second élément (12; 94) délimitant un espace (22; 96) dans lequel se trouve un tampon filtrant (32; 97) de matériau adsorbant les gaz, le second élément ayant une paroi de base (28; 100) et étant percé d'une ou de plusieurs petites ouvertures (30; 102) par lesquelles les gaz filtrés peuvent sortir, caractérisée par des couches respectives d'adhésif (36; 110) recouvertes chacune d'une couche protectrice pelable prévue sur la surface sensiblement plate (18; 114) du premier élément (10; 90) et sur une surface plate surmontant le second élément (12; 94), sauf dans la zone qui se trouve dans l'axe de ladite pointe (16; 108) quand on replie l'agrafe dans sa position fermée.

2. Agrafe selon la revendication 1, dans laquelle lesdits premier et second éléments sont moulés avec une charnière d'une seule pièce (14; 92) dans une matière plastique synthétique moulable.

3. Agrafe selon la revendication 2, dans laquelle ledit second élément (12) comprend une paroi périphérique (20) ayant un rebord d'une seule pièce (24) qui présente une surface plate (26) tournée vers le haut et la paroi de base (28) d'une seule pièce avec ladite paroi périphérique, délimitant ainsi l'espace (22) dans lequel se trouve un tampon filtrant (32) de matériau adsorbant les gaz, ladite paroi de base étant percée d'une ou de plusieurs petites ouvertures (30) par lesquelles les gaz filtrés peuvent s'échapper.

4. Agrafe selon la revendication 3, dans laquelle une couche de matière à porosité contrôlée est interposée entre la partie supérieure dudit tampon filtrant et ladite couche d'adhésif.

5. Agrafe selon la revendication 2, dans laquelle ledit second élément (94) comprend une paroi supérieure (116) percée d'une ouverture centrale (118) qui se trouve dans l'axe de la pointe (108) dudit premier élément (90) quand on replie l'agrafe dans sa position fermée, une paroi latérale (101) faisant corps avec ladite paroi supérieure et descendant du pourtour de ladite paroi supérieure, et un couvercle (100) percé d'une ou de plusieurs ouvertures relativement petites (102), ledit couvercle étant relié d'une seule pièce par une charnière (98) à une partie de ladite paroi latérale de telle sorte que ledit couvercle peut être encliqueté dans une position verrouillée contre la partie opposée (106) de ladite paroi latérale, délimitant ainsi un espace (96) entre ladite paroi supérieure, ladite paroi latérale et ledit couvercle; un tampon filtrant (97) de matériau adsorbant les gaz, placé dans ledit espace; ledit premier élément étant relié à ladite paroi supérieure du second élément par la charnière d'une seule pièce (92), et la couche d'adhésif (110) étant recouverte d'une couche protectrice pelable (112) qui surmonte ladite paroi supérieure, sauf dans la zone de ladite ouverture de la paroi supérieure.

6. Agrafe selon l'une quelconque des revendications précédentes, dans laquelle une couche (99) de matière à porosité contrôlée est interposée entre ledit tampon filtrant et ladite paroi supérieure du second élément.

7. Agrafe selon l'une quelconque des revendications précédentes, dans laquelle ledit tampon filtrant contient du charbon actif.

8. Poche de stomie comprenant des parois antérieure et postérieure de matière plastique synthétique (76) soudées l'une à l'autre le long d'au moins une partie notable de leurs bords périphériques, une ouverture ménagée dans ladite paroi antérieure de la poche, par laquelle l'anus artificiel peut faire saillie, la poche ayant une région supérieure de poche au-dessus de l'ouverture dudit anus artificiel, délimitée par une ligne de jonction discontinue (74) en forme d'arc de cercle qui relie lesdites parois antérieure et postérieure de la poche, ladite ligne de jonction étant telle qu'elle permet aux gaz de franchir la ligne de jonction à travers plusieurs brèches (78) qui y sont ménagées, mais telle qu'elle empêche l'écoulement de matière fécale, ladite ligne de jonction discontinue étant formée par thermo- soudage des parois antérieure et postérieure de la poche, caractérisé en ce que de courtes barres de soudure (80) sont situées à proximité et en regard de chaque brèche de façon à former en outre un trajet labyrinthique pour les gaz qui passent de la partie inférieure de ladite poche dans ladite région supérieure de la poche.

9. Poche de stomie selon la revendication 8, dans laquelle un raccord en forme de U (82) est placé sur la paroi antérieure de la poche entourant l'ouverture dudit anus artificiel.

10. Poche de stomie comportant une agrafe de filtre repliable qui lui est fixée, ladite poche de stomie comprenant des parois antérieure et postérieure de matière plastique synthétique soudées l'une à l'autre le long d'au moins une partie notable de leurs bords périphériques, une ouverture ménagée dans ladite paroi antérieure de la poche, par laquelle l'anus artificiel peut faire saillie, la poche ayant une région supérieure de poche, au-dessus de l'ouverture dudit anus artificiel, délimitée par une ligne de jonction discontinue (74) qui relie lesdites parois antérieure et postérieure de la poche, ladite ligne de jonction étant telle qu'elle permet aux gaz de franchir la ligne de jonction à travers une ou plusieurs brèches (78) qui y sont ménagées, mais telle qu'elle empêche l'écoulement de matière fécale; une agrafe de filtre repliable (72) fixée à la poche de stomie dans la région supérieure de la poche, ladite agrafe de filtre comprenant un premier et un second éléments qui sont réunis l'un à l'autre ou articulés l'un sur l'autre et qui sont faits d'un matériau pouvant être aisément plié par la pression des doigts d'une personne, ledit premier élément comportant une point de perçage de la poche, ledit second élément comportant une paroi de base et étant percé d'une ou de plusieurs petites ouvertures par lesquelles les gaz filtrés peuvent s'échapper et délimitant un espace dans lequel se trouve un tampon filtrant de matériau adsorbant les gaz, caractérisée en ce que la pointe dépasse à l'extérieur d'une surface sensiblement plate du premier élément et comporte un adhésif recouvert d'une couche protectrice pelable sur ladite surface plate et par une couche d'adhésif recouverte d'une couche protectrice pelable surmontant ledit second élément, sauf dans la zone qui se trouve dans l'axe de ladite pointe quand on replie l'agrafe dans sa position fermée, l'agencement étant tel que l'on fixe ladite agrafe de filtre à ladite région supérieure de la poche en retirant lesdites couches protectrices pelables, en plaçant ladite agrafe de filtre dans ladite région supérieure de la poche et, en un seul temps, en pliant et comprimant ladite agrafe de filtre de manière qu'un contact adhésif soit assuré entre lesdites parois antérieure et postérieure de la poche et lesdits premier et second éléments de l'agrafe, tandis qu'en même temps ladite pointe vient percer les deux dites parois de la poche.

*FIG.1*

*FIG.2*

*FIG.3*

*FIG.4*

*FIG.5*

FIG.6

FIG.7

FIG.8

FIG.9

FIG.10

FIG.11

FIG.12